# EUROPEAN PATENT SPECIFICATION

(11) **EP 2 682 148 B1**
(45) Date of publication and mention of the grant of the patent: **21.06.2017**
(21) Application number: 13185978.7
(22) Date of filing: 01.12.2009
(51) Int. Cl.: A61M 16/06, A62B 17/04, A41D 13/11

(54) **Hood for non-invasive ventilation of patients**
Haube zur nichtinvasiven Beatmung von Patienten
Cloche de Hood pour ventilation non invasive de patients

(30) Priority: 03.12.2008 IT MI20080396 U
(43) Date of publication of application: 08.01.2014
(62) Divisional of application: 09796987.7
(73) Proprietor: Intersurgical S.P.A., 41037 Mirandola, Modena (IT)
(72) Inventor: Navalesi, Paolo, 28100 Novara, Italy (IT); Olivieri, Carlo, 28100 Novara, Italy (IT); Rossi, Paolo, 41037 Mirandola (Modena) (IT)
(74) Representative: Corradini, Corrado

(56) References cited:
- EP-A1- 1 852 137
- EP-A2- 1 279 411
- WO-A2-03/097145
- WO-A2-2007/128571
- US-A- 4 620 538
- US-A- 5 819 728
- US-B1- 6 854 459

## Description

### Technical Field

The present invention relates to a hood for non-invasive ventilation of patients.

### Background Art

As is known, non-invasive ventilation of patients in general already uses hoods which, generally speaking, are provided by a substantially cylindrical container body with or without an upper cushion; the inlet and outlet connectors for the air and oxygen are connected to said body, and the hood is provided with a collar constituted by a thin membrane made of elastically flexible plastic material, which mates with the neck of the patient in order to provide the seal.

Such collar is typically provided by an extremely flexible film that adheres to the skin of the patient without applying pressure. Examples of known hoods according to the preamble of claim 1 are shown in WO 2007/128571, US 5 819 728 and US 6 854 459. Such flexibility and elasticity which, on the one hand, is particularly useful for providing the seal, on the other hand, constitutes a technical limitation of the hood, since it can reduce the effectiveness of the ventilation due to the fact that the collar is deformed elastically and raises the hood, requiring the physician to set therapies with higher pressure values.

Moreover, it can lead to a lack of synchronization during the beginning of the spontaneous respiratory act of the patient and the response of the ventilator, which is essential for correct ventilation therapy.

This aspect therefore influences directly the functionality of the hood, with the need to provide armpit restraining systems, such as for example ordinary padded straps designed to keep the hood in position.

The alternating swelling of the soft collar inevitably causes the upward movement of the hood, and therefore a traction is applied which constitutes a pressure of the straps under the patient's armpits, which in the long term inevitably inconveniences the patient.

### Disclosure of the Invention

The aim of the invention is to solve the problem described above, by providing a hood for non-invasive ventilation of patients that allows to providing a hood for non-invasive ventilation of patients that allows to reduce drastically the effect of the elasticity or compliance caused by the flexible collar, further achieving a reduction in the movement of the hood during the respiratory act.

Within this aim, an object of the invention is to provide an increase in the stiffness of the structure of the hood, so that it can be far less influenced by the sudden pressure variations that occur within the respiration circuit.

Another object of the present invention is to obtain a correct synchronization between the beginning of the spontaneous respiratory act of the patient and the response of the ventilator, thus obtaining a correct ventilation therapy.

Another object of the present invention is to provide a hood which, thanks to its particular constructive characteristics, is capable of giving the greatest assurances of reliability and safety in use.

This aim and these other objects which will become better apparent hereinafter are achieved by a hood for non-invasive ventilation of patients, according to the invention, comprising a containment body provided with at least one optically transparent portion and with a collar that can be mated hermetically to the neck of the patient, whose head is accommodated within said containment body, characterized in that it comprises a cushion and an annular body which can be associated around the neck and can be fixed to said containment body, said annular body fixed to said containment body being substantially rigid and adapted to act as an abutment element, together with the cushion, for said collar.

### Brief Description of the Drawings

Further characteristics and advantages of the present invention will become better apparent from the description of a preferred but not exclusive embodiment of a hood for non-invasive ventilation of patients, illustrated by way of non-limiting example in the accompanying drawings, wherein:
Figure 1 is a schematic exploded and partially sectional view of the hood, with an annular body provided by two hinged elements;
Figure 2 is a schematic view of the hood with an inner inflatable cushion which is applied or heat-sealed to the annular body;
Figure 3 is a view of the hood in the position for use;
Figure 4 is a bottom plan view of the hood;

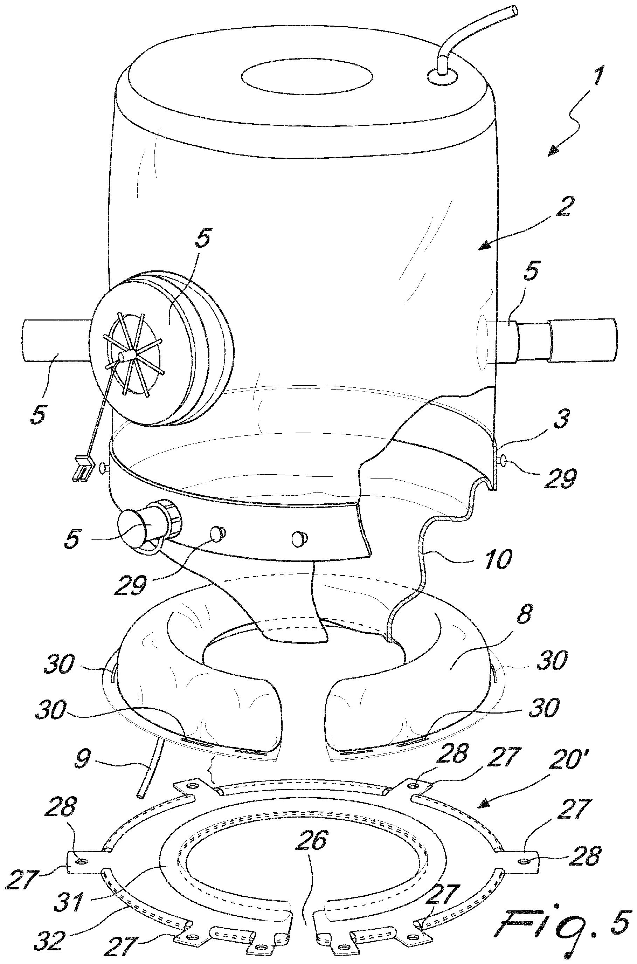

Figure 5 is an exploded view of the hood with an annular body provided in one piece;

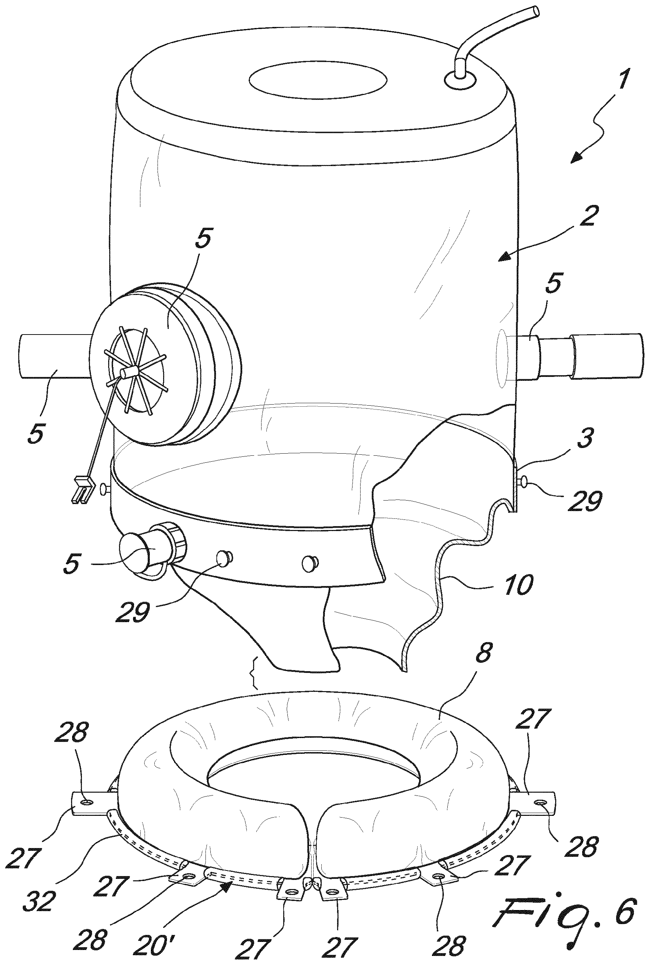

Figure 6 is a view of the lower cushion applied or heat-sealed to the monolithic annular body; and

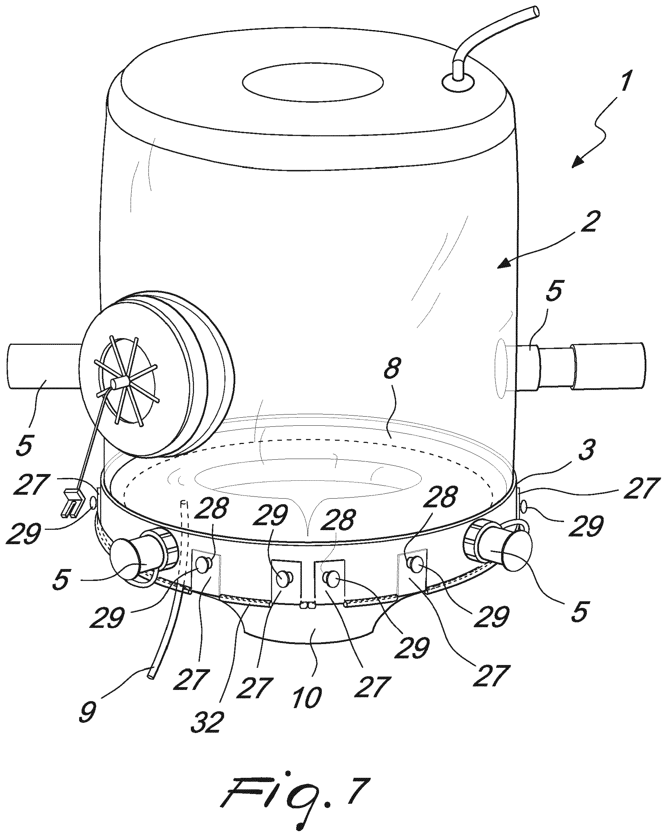

Figure 7 is a view of the hood with the monolithic annular body shown assembled.

### Ways of carrying out the Invention

With reference to the figures, the hood for non-invasive ventilation of patients in general, generally designated by the reference numeral 1, comprises a containment body 2, which is advantageously provided by a cylindrical element made of optically transparent material, which though being flexible cannot expand. The containment body 2, at its lower open end, is advantageously connected to a rigid base ring 3, to which a collar 10 is connected which advantageously is made of flexible material in order to be mated hermetically to the neck of the patient, whose head is accommodated in the containment body 2.

As is common practice, the containment body is provided with intake and discharge connectors and with optional openings which can be closed for access to the interior and so forth, all generically designated by the reference numeral 5.

Moreover an inner cushion is provided, generally designated by the reference numeral 8, which can be inflated with an external connector 9, which is positioned below the collar 10, fixed or heat-sealed on the annular body 20, moreover, the cushion 8 can be made of an inflatable material or can be made of a soft material fixed or heat-sealed to the annular body.

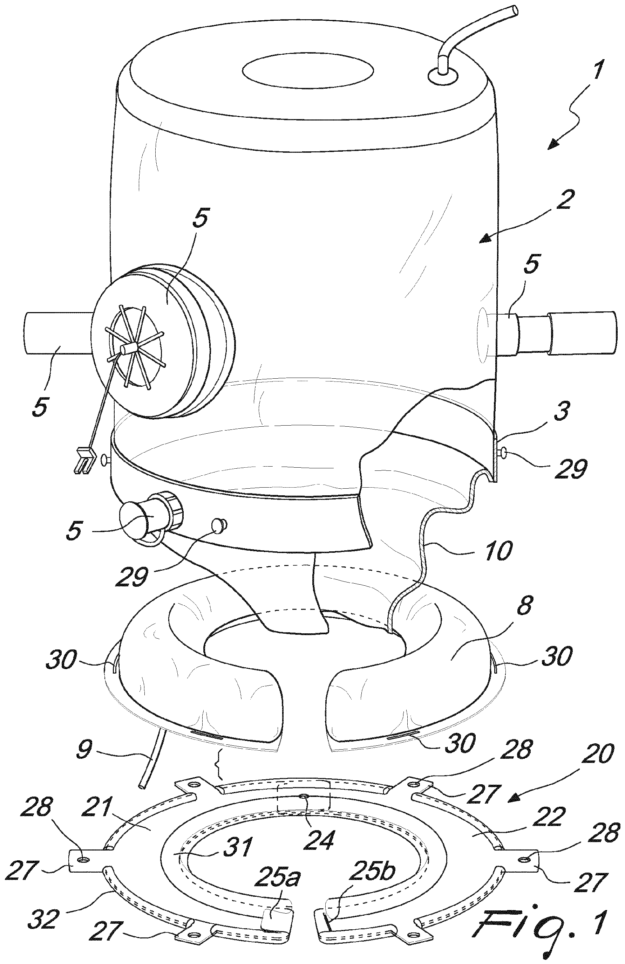

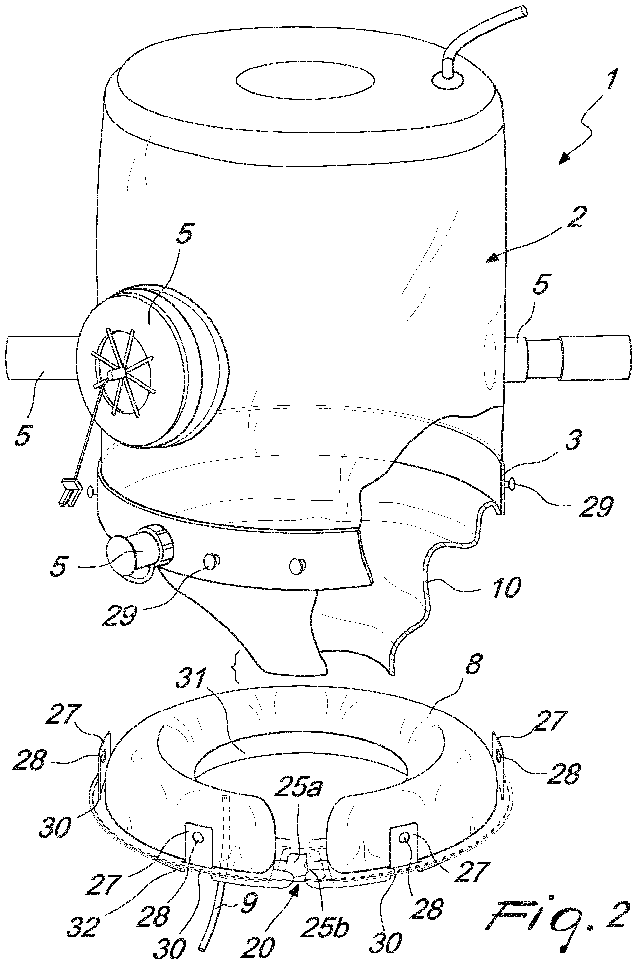

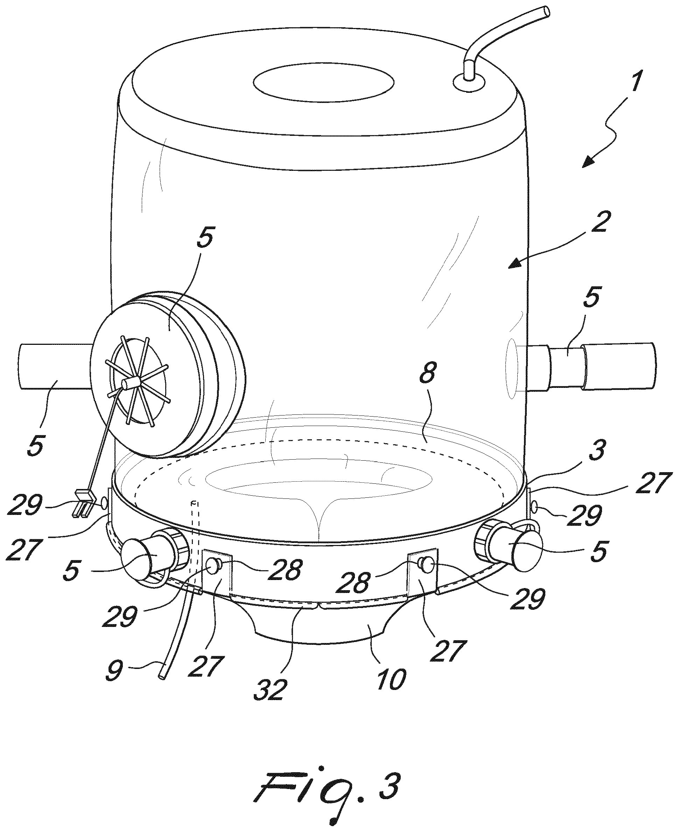

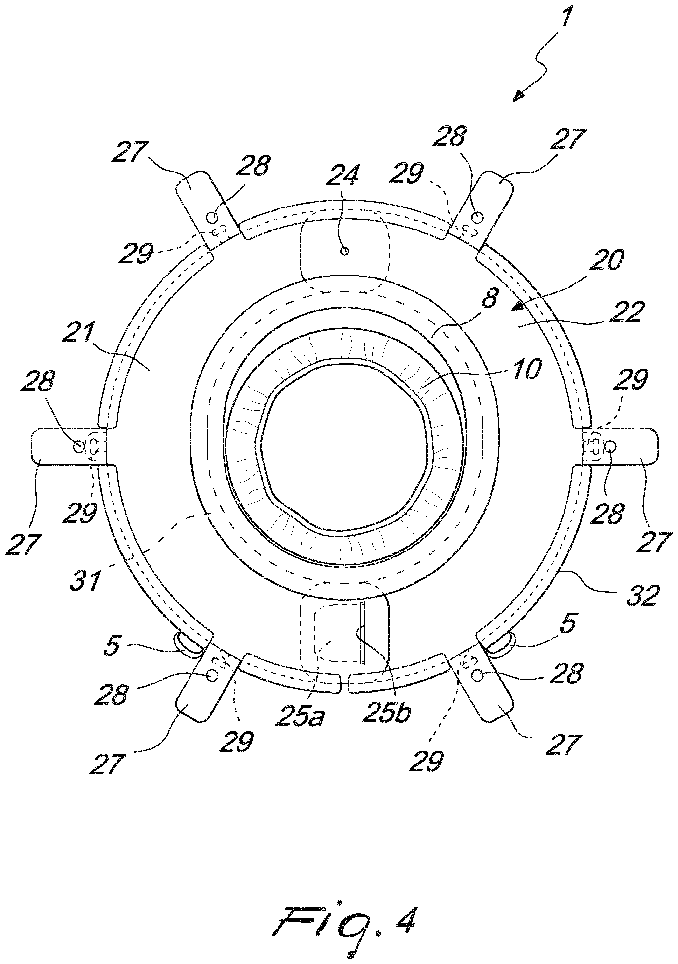

The peculiarity of the invention consists in that an annular body, designated by the reference numeral 20, is provided which, in the embodiment shown in Figures 1 to 4, is constituted by two semicircles 21 and 22, which are connected one another by pivoting means constituted by a pivoting pin 24 and have, at the other end, joining means constituted by a tab 25a arranged on one end which can be inserted in a slot 25b arranged on the facing end.

The annular body can be provided by means of substantially rigid elements or optionally by means of elements that have a certain flexibility and are advantageously provided by means of a sheet made of plastic material such as polyvinyl chloride, polypropylene, acrylonitrile-butadienestyrene, polyoxymethylene, known commercially by the trade name Delrin, or other polymers.

The annular body 20 is provided with means for fixing to the containment body 2, which in a possible embodiment are constituted by radial tabs 27, which are fixed to or heat-sealed or are part of the annular body 20 and have a through hole 28 to provide a stable connection with coupling pins 29 provided at the base of the containment body at the rigid ring 3.

With the flexible element that provides the annular body 20 mated to the containment body, the annular body is substantially rigid and is adapted to act as an abutment element for the collar 10 together with the interposed cushion 8.

According to an embodiment that is linked conceptually to the preceding one, the annular body, designated by the reference numeral 20', can be provided in one piece with a notch 26 which can be closed, with the possibility to arrange the annular body around the neck of the patient by utilizing the flexibility of the material.

The tabs 27 can enter coupling slots 30 provided on the cushion 8, or optionally the cushion 8 can be directly heat-sealed or mated on the annular body 20 or 20'.

Moreover, on the inner edge of the annual body 20 or 20', either in two parts or in one piece, there is a soft protective edge 31; optionally, on the outer edge there is an outer protective edge 32.

With the described arrangement, when the annular body is connected stably to the containment body, the collar 10 abuts against the cushion 8 and the abutment plane constituted by the annular body 20, thus obtaining, in a way, a stiffening of the structure of the hood, which prevents the elastic deformation of the collar and consequently prevents the creation both of the upward displacement of the hood and the variation of the inner volume of the containment body, both of which are due to the elastic deformation of the collar.

In practice the collar is retained between the inner cushion 8 and the abutment plane 20, thus providing an optimum seal on the neck of the patient without however causing its elastic deformation, with consequent negative influence on the pressure values during the respiratory act.

From what has been described above it is evident that the invention achieves the proposed aim and objects, and in particular the fact is stressed that the presence of an annular body constituted by an element that is rigid or optionally becomes rigid thanks to its connection to the containment body provides an abutment element for the flexible collar, thus allowing to obtain optimum sealing values without however having the corresponding deformation of the collar, which accordingly does not cause the rise of the hood or the variation of the inner volume of the containment body, with the consequent possibility to avoid using the armpit straps to retain the hood.

The invention thus conceived is susceptible of numerous modifications and variations, all of which are within the scope of the appended claims.

All the details may further be replaced with other technically equivalent elements.

In practice, the materials used, so long as they are compatible with the specific use, as well as the contingent shapes and dimensions, may be any according to requirements and to the state of the art. For example the disclosures in the Utility Model Application No. MI2008U000396

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A hood for non-invasive ventilation of patients, comprising a flexible containment body (2) provided with at least one optically transparent portion and with an elastically flexible collar (10) that can be mated hermetically to the neck of the patient whose head is accommodated within said containment body (2), wherein the hood comprises
- an annular body (20, 20') which can be associated around the neck and can be fixed to said containment body (2), said annular body (20, 20') fixed to said containment body being rigid,
- a cushion (8) which can be associated around the neck and can be fixed to said containment body (2),
wherein said annular body (20,20') is adapted to act as an abutment element, together with the cushion (8), for said collar (10),
**characterized in that** the cushion (8) is interposed between the collar (10) and the abutment plane of said annular body (20,20').

2. A hood for non-invasive ventilation of patients, comprising a flexible containment body (2) provided with at least one optically transparent portion and with an elastically flexible collar (10) that can be mated hermetically to the neck of the patient whose head is accommodated within said containment body (2), wherein the hood comprises
- an annular body (20, 20') which can be associated around the neck and can be fixed to said containment body (2), said annular body (20, 20') fixed to said containment body being rigid,
- a cushion (8) which can be associated around the neck and can be fixed to said containment body (2),
wherein said annular body (20,20') is adapted to act as an abutment element, together with the cushion (8), for said collar (10),
**characterized in that** the collar (10) is retained between the cushion (8) and the abutment plane of said annular body (20,20').

3. The hood according to one or more of the preceding claims, **characterized in that** said annular body (20) is constituted by two semicircles (21, 22), which are connected one another at one end and are provided at the other end with joining means.

4. The hood according to the preceding claims, **characterized in that** said semicircles (21, 22) are substantially rigid.

5. The hood according to one or more of the preceding claims, **characterized in that** said two semicircles (21, 22) are made of substantially flexible material, adapted to be stiffened by the connection to said containment body (2).

6. The hood according to one or more of the preceding claims from 3 to 5, **characterized in that** said joining means are constituted by a tab (25a), which is arranged at one end of one of said semicircles (21, 22) and can be inserted in a slot (25b) arranged on a facing end of the other semicircle (21, 22).

7. The hood according to one or more of the preceding claims from 8 and 9, **characterized in that** said substantially flexible semicircles (21, 22) are made of a sheet of plastic material.

8. The hood according to one or more of the preceding claims, **characterized in that** it comprises fixing means (27, 28 and 29) for fixing said annular body (20, 20') to said containment body (2).

9. The hood according to claim 8, **characterized in that** said fixing means are constituted by radial tabs (27) that protrude from said annular body (20, 20') and are provided with a through hole (28) for connection to a corresponding coupling pin (29) which is provided correspondingly at a rigid ring (3) arranged at the base of said containment body (2).

10. The hood according to claim 9, **characterized in that** it comprises at least one inner cushion (8), which can engage at least one face of said collar (10), said at least one cushion (8) being provided with coupling slots (30) for the passage of said radial tabs (27).

11. The hood according to one or more of the preceding claims, **characterized in that** it comprises at least one inner cushion (8), which can engage at least one face of said collar (10) and is jointly associated with said annular body (20, 20').

12. The hood according to one or more of the preceding claims, **characterized in that** said annular body (20') is constituted by a single piece provided with a notch (26).

13. The hood according to one or more of the preceding claims, **characterized in that** it comprises, on the inner edge of said annular body (20, 20'), a soft protective edge (31).

## Patentansprüche

1. Haube zur nichtinvasiven Beatmung von Patienten, umfassend einen flexiblen Einschließungskörper (2), der mit mindestens einem optisch transparenten Abschnitt und einer elastisch-flexiblen Manschette (10) versehen ist, die hermetisch an den Hals des Patienten angepasst werden kann, dessen Kopf in dem Einschließungskörper (2) untergebracht ist, wobei die Haube Folgendes umfasst:
einen ringförmigen Körper (20, 20'), der um den Hals des Patienten herum angebracht und am Einschließungskörper (2) befestigt werden kann, wobei der am Einschließungskörper befestigte ringförmige Körper (20, 20') starr ist,
ein Polster (8), das um den Hals des Patienten herum angebracht und am Einschließungskörper (2) befestigt werden kann,
wobei der ringförmige Körper (20, 20') dafür eingerichtet ist, zusammen mit dem Polster (8) als ein Auflageelement für die Manschette (10) zu dienen,
**dadurch gekennzeichnet, dass** das Polster (8) zwischen der Manschette (10) und der Auflageebene des ringförmigen Körpers (20, 20') eingeschoben ist.

2. Haube zur nichtinvasiven Beatmung von Patienten, umfassend einen flexiblen Einschließungskörper (2), der mit mindestens einem optisch transparenten Abschnitt und einer elastisch-flexiblen Manschette (10) versehen ist, die hermetisch an den Hals des Patienten angepasst werden kann, dessen Kopf in dem Einschließungskörper (2) untergebracht ist, wobei die Haube Folgendes umfasst:
einen ringförmigen Körper (20, 20'), der um den Hals des Patienten herum angebracht und am Einschließungskörper (2) befestigt werden kann, wobei der am Einschließungskörper befestigte ringförmige Körper (20, 20') starr ist,
ein Polster (8), das um den Hals des Patienten herum angebracht und am Einschließungskörper (2) befestigt werden kann,
wobei der ringförmige Körper (20, 20') dafür eingerichtet ist, zusammen mit dem Polster (8) als ein Auflageelement für die Manschette (10) zu dienen,
**dadurch gekennzeichnet, dass** das Polster (8) zwischen der Manschette (10) und der Auflageebene des ringförmigen Körpers (20, 20') gehalten wird.

3. Haube nach einem oder mehreren der vorhergehenden Ansprüche, **dadurch gekennzeichnet, dass** der ringförmige Körper (20) von zwei Halbkreisen (21, 22) gebildet ist, die an einem Ende miteinander und am anderen Ende mit Verbindungsmitteln verbunden sind.

4. Haube nach den vorhergehenden Ansprüchen, **dadurch gekennzeichnet, dass** die Halbkreise (21, 22) im Wesentlichen starr sind.

5. Haube nach einem oder mehreren der vorhergehenden Ansprüche, **dadurch gekennzeichnet, dass** die zwei Halbkreise (21, 22) aus einem im Wesentlichen flexiblen Material bestehen, das dafür eingerichtet ist, durch die Verbindung mit dem Einschließungskörper (2) versteift zu werden.

6. Haube nach einem oder mehreren der vorhergehenden Ansprüche von 1 bis 5, **dadurch gekennzeichnet, dass** die Verbindungsmittel von einer Lasche (25a) gebildet sind, die an einem Ende eines der Halbkreise (21, 22) angeordnet ist und in einen Schlitz (25b) eingeführt werden kann, der an einem gegenüberliegenden Ende des anderen Halbkreises (21, 22) angeordnet ist.

7. Haube nach einem oder mehreren der vorhergehenden Ansprüche von 8 und 9, **dadurch gekennzeichnet, dass** die im Wesentlichen flexiblen Halbkreise (21, 22) aus einer Bahn aus Kunststoff bestehen.

8. Haube nach einem oder mehreren der vorhergehenden Ansprüche, **dadurch gekennzeichnet, dass** sie Befestigungsmittel (27, 28 und 29) zum Befestigen des ringförmigen Körpers (20, 20') am Einschließungskörper (2) umfasst.

9. Haube nach Anspruch 8, **dadurch gekennzeichnet, dass** die Befestigungsmittel von radialen Laschen (27) gebildet sind, die aus dem ringförmigen Körper (20, 20') hervorstehen und mit einer Durchgangsöffnung (28) für die Verbindung mit einem entsprechenden Kopplungsstift (29) versehen sind, der entsprechend an einem starren Ring (3) bereitgestellt ist, der am Unterteil des Einschließungskörpers (2) angeordnet ist.

10. Haube nach Anspruch 9, **dadurch gekennzeichnet, dass** sie mindestens ein inneres Polster (8) umfasst, das mit mindestens einer Fläche der Manschette (10) in Eingriff gelangen kann, wobei das mindestens eine Polster (8) mit Kopplungsschlitzen (30) für den Durchlass der radialen Laschen (27) versehen ist.

11. Haube nach einem oder mehreren der vorhergehenden Ansprüche, **dadurch gekennzeichnet, dass** sie mindestens ein inneres Polster (8) umfasst, das mit mindestens einer Fläche der Manschette (10) in Eingriff gelangen kann und gemeinsam mit dem ringförmigen Körper (20, 20') verbunden ist.

12. Haube nach einem oder mehreren der vorhergehenden Ansprüche, **dadurch gekennzeichnet, dass** der ringförmige Körper (20') von einem einzelnen Stück gebildet ist, das mit einer Nut (26) versehen ist.

13. Haube nach einem oder mehreren der vorhergehenden Ansprüche, **dadurch gekennzeichnet, dass** sie am Innenrand des ringförmigen Körpers (20, 20') einen weichen Schutzrand (31) umfasst.

## Revendications

1. Casque de ventilation non invasive de patients, comprenant un corps de confinement flexible (2) muni d'au moins une portion optiquement transparente et d'un collier flexible élastiquement (10) qui peut être adapté hermétiquement au cou du patient dont la tête est logée à l'intérieur dudit corps de confinement (2), dans lequel le casque comprend
- un corps annulaire (20, 20') qui peut être associé autour du cou et peut être fixé audit corps de confinement (2), ledit corps annulaire (20, 20') fixé audit corps de confinement étant rigide,
- un coussin (8) qui peut être associé autour du cou et peut être fixé audit corps de confinement (2), dans lequel ledit corps annulaire (20, 20') est adapté pour agir comme un élément de butée, conjointement au coussin (8), pour ledit collier (10),
**caractérisé en ce que** le coussin (8) est interposé entre le collier (10) et le plan de butée dudit corps annulaire (20, 20').

2. Casque de ventilation non invasive de patients, comprenant un corps de confinement flexible (2) muni d'au moins une portion optiquement transparente et d'un collier flexible élastiquement (10) qui peut être adapté hermétiquement au cou du patient dont la tête est logée à l'intérieur dudit corps de confinement (2), dans lequel le casque comprend
- un corps annulaire (20, 20') qui peut être associé autour du cou et peut être fixé audit corps de confinement (2), ledit corps annulaire (20, 20') fixé audit corps de confinement étant rigide,
- un coussin (8) qui peut être associé autour du cou et peut être fixé audit corps de confinement (2), dans lequel ledit corps annulaire (20, 20') est adapté pour agir comme un élément de butée, conjointement au coussin (8), pour ledit collier (10),
**caractérisé en ce que** le collier (10) est retenu entre le coussin (8) et le plan de butée dudit corps annulaire (20, 20').

3. Casque selon une ou plusieurs des revendications précédentes, **caractérisé en ce que** ledit corps annulaire (20) est constitué de deux demi-cercles (21, 22), qui sont connectés l'un à l'autre à une extrémité et sont munis à l'autre extrémité de moyens de jonction.

4. Casque selon les revendications précédentes, **caractérisé en ce que** lesdits demi-cercles (21, 22) sont sensiblement rigides.

5. Casque selon une ou plusieurs des revendications précédentes, **caractérisé en ce que** lesdits deux demi-cercles (21, 22) sont réalisés en matériau sensiblement flexible, adapté pour être raidi par la connexion avec ledit corps de confinement (2).

6. Casque selon une ou plusieurs des revendications précédentes 3 à 5, **caractérisé en ce que** lesdits moyens de jonction sont constitués par une languette (25a), qui est disposée à une extrémité d'un desdits demi-cercles (21, 22) et peut être insérée dans une fente (25b) disposée sur une extrémité faisant face de l'autre demi-cercle (21, 22).

7. Casque selon une ou plusieurs des revendications précédentes 8 et 9, **caractérisé en ce que** lesdits demi-cercles sensiblement flexibles (21, 22) sont constitués d'une feuille de matériau plastique.

8. Casque selon une ou plusieurs des revendications précédentes, **caractérisé en ce qu'il** comprend des moyens de fixation (27, 28 et 29) pour fixer ledit corps annulaire (20, 20') audit corps de confinement (2).

9. Casque selon la revendication 8, **caractérisé en ce que** lesdits moyens de fixation sont constitués par des languettes radiales (27) qui font saillie à partir dudit corps annulaire (20, 20') et sont pourvues d'un trou passant (28) pour la connexion à une broche d'accouplement correspondante (29) qui est disposée en correspondance d'un anneau rigide (3) agencé à la base dudit corps de confinement (2).

10. Casque selon la revendication 9, **caractérisé en ce qu'il** comprend au moins un coussin intérieur (8), qui peut engager au moins une face dudit collier (10), ledit au moins un coussin (8) étant pourvu de fentes d'accouplement (30) pour le passage desdites languettes radiales (27).

11. Casque selon une ou plusieurs des revendications précédentes, **caractérisé en ce qu'il** comprend au moins un coussin intérieur (8), qui peut engager au moins une face dudit collier (10) et est associé solidairement avec ledit corps annulaire (20, 20').

12. Casque selon une ou plusieurs des revendications précédentes, **caractérisé en ce que** ledit corps annulaire (20') est constitué par une unique pièce munie d'une encoche (26).

13. Casque selon une ou plusieurs des revendications précédentes, **caractérisé en ce qu'il** comprend, sur le bord intérieur dudit corps annulaire (20, 20'), un bord protecteur moelleux (31).
